# EUROPÄISCHE PATENTSCHRIFT

(11) **EP 1 568 349 B1**
(45) Veröffentlichungstag und Bekanntmachung des Hinweises auf die Patenterteilung: **08.04.2015**
(21) Anmeldenummer: 05003217.6
(22) Anmeldetag: 16.02.2005
(51) Int. Cl.: A61K 8/37, A61K 8/39, A61K 8/891, A61K 8/894, A61K 8/44, A61K 8/73, A61Q 5/12

(54) **Konditionierende Reinigungszubereitung für die Haare auf Basis von Silikonen und bestimmten Wachsen**
Conditioning cleansing composition for the hair based on silicones and specific waxes
Composition conditionnante nettoyante pour les cheveux à base de silicones et de cires particulières

(30) Priorität: 24.02.2004 DE 102004009426
(43) Veröffentlichungstag der Anmeldung: 31.08.2005
(73) Patentinhaber: Cognis IP Management GmbH, 40589 Düsseldorf (DE)
(72) Erfinder: Nieendick, Claus, 47807 Krefeld (DE); Becker, Anke, 40468 Düsseldorf (DE); Seipel, Werner, 40723 Hilden (DE)

(56) Entgegenhaltungen:
- EP-A- 1 329 215
- EP-A1- 1 510 199
- WO-A-2004/014326
- FR-A- 2 802 087
- US-A- 6 004 544
- US-A1- 2003 147 842
- US-B1- 6 297 203
- US-B1- 6 482 418

## Beschreibung

### Gebiet der Erfindung

Gegenstand der vorliegenden Erfindung sind spezielle konditionierende Reinigungszubereitungen auf Basis von Silikonen und bestimmten Wachsen für die Reinigung von keratinischen Fasern.

### Stand der Technik

Es ist bekannt, dass Haare nach dem Waschen nicht nur stumpf aussehen, sondern sich auch sehr schlecht kämmen und frisieren lassen. Daher werden modernen Shampoozubereitungen Konditioniermittel zugesetzt, bei denen diese Nachteile nicht auftreten sollen. So beschreibt die EP 0 918 506 B1 kosmetische Zubereitungen enthaltend bestimmte langkettige Dialkylether in Kombination mit Silikonen in Konditioniershampoos. Gegenstand der WO 97/47274 sind ebenfalls kosmetische Reinigungszubereitungen, die als Perlglanzmittel eine Kombination von bestimmten langkettigen Dialkylethern oder Dialkylcarbonaten mit Tensiden und Polyolen enthalten. Auch die WO 98/20845 hat Perlglanzkonzentrate zum Gegenstand, die Dialkylether in Kombination mit Silikonen und bestimmten Emulgatoren enthalten. Aus der WO 99/06414 sind Zubereitungen bekannt, die auf Basis von alkoxylierten Dialkylethern, Tensiden und Silikonen hergestellt werden.

Die Aufgabe der vorliegenden Erfindung hat nun darin bestanden, kosmetische Zubereitungen für die Reinigung von Haaren bereitzustellen, die in hohem Maße die Kämmarbeit nach dem Waschen reduzieren und die Haare konditionieren, gleichzeitig aber über ein hohes Schaumvermögen und eine gute Reinigungsleistung zu verfügen. Auch sollen in die Zubereitungen nicht lösliche Silikone stabil eingearbeitet werden können.

### Beschreibung der Erfindung

Es wurde gefunden, dass kosmetische Zubereitungen enthaltend bestimmte Wachse, Silikone, amophotere und/oder zwitterionische Tenside und kationische Polymere die Kämmarbeit an Haaren um mindestens 25 % erniedrigen. Die Kombination dieser bestimmten Wachse, Silikone und kationischer Polymere entfaltet eine synergistische Wirkung, die die Kämmarbeit in einem wesentlich höherem Maße erniedrigt als dies durch die Wirkung der einzelnen Komponenten zu erwarten gewesen wäre.

Gegenstand der vorliegenden Erfindung ist daher eine kosmetische Zubereitung enthaltend
(a) mindestens einen Wachs ausgewählt aus der Gruppe, die gebildet wird von Dialkyl(en)alkoxyethern der Formel (1)

   R¹-(OCₙH₂ₙ)ₓ-O-(CₘH₂ₘO)_{y}-R² (I)

   in der R¹ und R² unabhängig voneinander für Alkyl- und/oder Alkylenreste mit 16 bis 36 Kohlenstoffatomen, n und m unabhängig voneinander für Zahlen von 2 bis 4 und x und y unabhängig voneinander für Zahlen von 0 bis 10 stehen, wobei die Summe von x und y 1 bis 10 beträgt, und Dialkyl(en)carbonaten mit geradkettigen, verzweigten, gesättigten oder ungesättigten Alkylketten mit 16 bis 36 Kohlenstoffatomen sowie Mischungen hiervon,
(b) mindestens ein Silikon,
(c) mindestens ein amphoteres und/oder zwitterionisches Tensid und
(d) mindestens ein kationisches Polymer.

In einer besonders bevorzugten Ausführungsform enthält die erfindungsgemäße kosmetische Zubereitung
(a) 0,3 bis 5 Gew.% mindestens eines Wachses ausgewählt aus der Gruppe, die gebildet wird von Dialkyl(en)alkoxyethern der Formel (I),

   R¹-(OCₙH₂ₙ)ₓ-O-(CₘH₂ₘO)_{y}-R² (I)

   in der R1 und R2 unabhängig voneinander für Alkyl- und/oder Alkylenreste mit 16 bis 36 Kohlenstoffatomen, n und m unabhängig voneinander für Zahlen von 2 bis 4 und x und y unabhängig für Zahlen von 0 bis 10 stehen, wobei die Summe von x und y 1 bis 10 beträgt, und Dialkyl(en)carbonaten mit geradkettigen, verzweigten, gesättigten oder ungesättigten Alkylketten mit 16 bis 36 Kohlenstoffatomen sowie Mischungen hiervon,
(b) 0,2 bis 5 Gew.% mindestens eines Silikons,
(c) 1 bis 50 Gew.% mindestens eines amphoteren und/oder zwitterionischen Tensids und
(d) 0,1 bis 1 Gew.% mindestens eines kationischen Polymers.

### Dialkyl(en)alkoxyether

Bei den erfindungsgemäßen Dialkyl(en)alkoxyethern handelt es sich um Verbindungen der Formel (I)

R¹-(OCₙH₂ₙ)ₓ-O-(CₘH₂ₘO)_{y}-R² (I)

in der R¹ und R² unabhängig voneinander für Alkyl- und/ oder Alkenylreste mit 16 bis 36 Kohlenstoffatomen, vorzugsweise mit 18 bis 24 Kohlenstoffatomen, stehen, n und m unabhängig voneinander für Zahlen von 2 bis 4 und x und y unabhängig voneinander für Zahlen von 0 bis 10 stehen, wobei die Summe von x und y 1 bis 10 und in einer bevorzugten Ausführungsform 1 bis 4 beträgt. Dabei ist anzumerken, daß die Summe aus x und y einen Mittelwert darstellt, da nur mittlere Alkoxylierungsgrade der Alkyl(en)alkoxyether bestimmt werden können. Die erfindungsgemäßen Alkyl(en)alkoxyether liegen in engen Homologenverteilungen vor, da Alkyl(en)alkoxyether der genannten Art üblicherweise durch Kondensation der entsprechenden alkoxylierten Alkohole hergestellt werden.

Um zu den erfindungsgemäßen Alkyl(en)alkoxyethern zu gelangen, kann man beispielsweise Fettalkohole mit 16 bis 36 Kohlenstoffatomen, vorzugsweise 18 bis 24 Kohlenstoffatomen, alkoxylieren, vorzugsweise ethoxylieren und/oder propoxylieren, insbesondere bevorzugt werden die Fettalkohole ethoxyliert. Die Alkoxylierung der Fettalkohole kann beispielsweise derart erfolgen, dass sie in einer Random-Polymerisation mit Ethylenoxid und Propylenoxid umgesetzt werden, man kann jedoch auch beispielsweise EO/PO-Blockpolymere herstellen. Beide Arten alkoxylierter Verbindungen sollen im Rahmen der vorliegenden Erfindung umfasst sein. Bei der Alkoxylierung erhält man eine Homologenverteilungen, besonders bevorzugt werden alkoxylierte Fettalkohole mit enger Homologenverteilung zur Herstellung der erfindungsgemäßen Alkyl(en)alkoxyether eingesetzt. Bei anschließender saurer oder basischer Kondensation der entsprechenden alkoxylierten Fettalkohole gelangt man zu den erfindungsgemäß einzusetzenden Alkyl(en)alkoxyethern. Diese umfassen sowohl symmetrische als auch unsymmetrische Alkyl(en)alkoxyether.

Alkyl(en)alkoxyether mit besonders vorteilhaften konditionierenden Eigenschaften werden durch Kondensation von Fettalkoholen mit 16 bis 36 Kohlenstoffatomen, wie beispielsweise Cetylalkohol, Cetearylalkohol, Stearylalkohol, Isostearylalkohol, Oleylalkohol, Behenylalkohol und/oder Erucylalkohol erhalten. Aufgrund des großen synergistischen Zusammenwirkens mit kationischen Polymeren und Silikonen in bezug auf eine sehr starke Erniedrigung der Kämmarbeit ist PEG-4 Distearylether besonders bevorzugt.

Die erfindungsgemäßen Zubereitungen enthalten die o.g. Alkyl(en)alkoxyether in Mengen von 0,3 bis 5 Gew.%, vorzugsweise 0,5 bis 2,5 Gew.% und in einer besonders bevorzugten Ausführungsform von 1 bis 2 Gew.%.

### Dialkyl(en)carbonate

Bei den erfindungsgemäßen Dialkyl(en)carbonaten handelt es sich um Verbindungen der Formel (II) R³O-CO-OR⁴ (II) in der R³ und R⁴ unabhängig voneinander für gegebenenfalls hydroxyfunktionalisierte Alkyl- und/oder Alkenylreste mit 16 bis 36 Kohlenstoffatomen stehen. Die Stoffe werden erhalten, indem man beispielsweise Dimethyl- oder Diethylcarbonat mit den entsprechenden Fettalkoholen bzw. Hydroxyalkoholen in an sich bekannter Weise umestert. Demzufolge können die Alkyl(en)carbonate symmetrisch oder unsymmetrisch aufgebaut sein. Vorzugsweise werden jedoch Carbonate eingesetzt, in denen R³ und R⁴ gleich sind und für Alkylreste mit 18 bis 24 Kohlenstoffatomen stehen. Besonders bevorzugt sind Umesterungsprodukte von Dimethyl- bzw. Diethylcarbonat mit Cetylalkohol, Cetearylalkohol, Stearylalkohol, Isostearylalkohol, Oleylalkohol, 12-Hydroxystearylalkohol, Behenylalkohol und/oder Erucylalkohol in Form ihrer Mono- und Diester bzw. deren technischen Mischungen.

In den erfindungsgemäßen Zubereitungen sind die Dialkyl(en)carbonate in Mengen von 0,3 bis 5 Gew.%, vorzugsweise von 0,5 bis 2,5 Gew.% und besonders bevorzugt von 1 bis 2 Gew.% enthalten.

### Amphotere bzw. zwitterionische Tenside

Als zwitterionische Tenside werden solche oberflächenaktiven Verbindungen bezeichnet, die im Molekül mindestens eine quartäre Ammoniumgruppe und mindestens eine Carboxylat-und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylamino-propyl-N,N-dimethylammoniumglycinate, beispielsweise das Kokosacylaminopropyl-dimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. In einer besonders bevorzugten Ausführungsform handelt es sich bei dem Tensid der Komponente (c) umd Cocamidopropylbetain. Ebenfalls geeignete Tenside sind ampholytische Tenside. Unter ampholytischen Tensiden werden solche oberflächenaktiven Verbindungen verstanden, die außer einer Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkyl-sarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Die amphoteren bzw. zwitterionischen Tenside werden in den erfindungsgemäßen Zubereitungen in Mengen von 1 bis 50 Gew.% , vorzugsweise von 5 bis 20 Gew.% und in einer besonders bevorzugten Auführungsform von 8 bis 15 Gew.% eingesetzt.

### Silikone

Geeignete Silikone im Sinne der vorliegenden Erfindung stellen Polyorganosiloxane dar, die sowohl flüssig als auch harzförmig vorliegen können. Vorzugsweise handelt es sich bei den Silikonen (b) um nichtflüchtige Verbindungen, die ausgewählt sind aus Polyalkylsiloxanen, Polyarylsiloxanen, Polyalkylarylsiloxanen, den Silikongummis und -harzen und organisch modifizierten Polyorganosiloxanen, sowie jedwede Mischung dieser Verbindungen.

Beipielhaft seien genannt: Polydimethylsiloxane mit endständigen Trimethylsilylgruppen, Polydimethylsiloxane mit endständigen Dimethylsilanolgruppen, Poly-(C₁-C₂₀)-alkylsiloxane, Polydimethylsiloxan/Methylvinylsiloxane, Polydimethylsiloxan/Diphenylsiloxan, Polydimethylsiloxan/Phenylmethylsiloxan, Polydimethylsiloxan/Diphenylsiloxan/ Methylvinylsiloxane, Gemische aus einem am Kettenende hydroxylierten Polydimethylsiloxan mit einem cyclischen Polydimethylsiloxan, Gemischen aus einem Polydimethylsiloxangummi und einem cyclischen Siloxan, Gemischen aus Polydimethylsiloxanen unterschiedlicher Viskosität, geradkettige und/oder verzweigte Polydimethylmethylphenylsiloxane und Polydimethyldiphenylsiloxane, Silikongummis auf Basis von Polydiorganosiloxanen mit einer Molmasse im Bereich von 2000000 bis 1000000 (einzeln oder in Form eines Gemisches in Lösungsmittel), chemisch modifizierte Polyorganosiloxane enhaltend folgende funktionelle Gruppen: Polyethylenoxy- und/oder Polypropylenoxy-Gruppen, gegebenenfalls substituierte Amingruppen, Thiolgruppen, Alkoxygruppen, Hydroxyalkylgruppen und Acyloxyalkylgruppen.

In den erfindungsgemäßen Zubereitungen werden die o.g. Silikone in Mengen von 0,2 bis 5 Gew.%, vorzugsweise von 0,5 bis 2,5 Gew.% eingesetzt.

### Kationische Polymere

Die erfindungsgemäßen Zubereitungen enthalten wenigstens ein kationisches Polymer in einer Menge von 0,1 bis 1 Gew.%, in einer besonders bevorzugten Auführungsform von 0,1 bis 0,5 Gew.%. Das kationische Polymer ist dabei vorzugsweise ausgewählt aus der Gruppe der kationisch modifizierten Polyacrylate, der kationisch modifizierten Polysaccharide, der kationisch modifizierten Polyacrylamide oder einem beliebigen Gemisch dieser Polymere. Besonders bevorzugt sind Polyquatemium-7, Polyquaternium-10 und kationischer Guar Gum, da insbesondere bei Verwendung dieser Polymere in den erfindungsgemäßen Zubereitungen das Haar nach dem Waschen ausgesprochen weich erscheint und während des Waschens ein feinblasiger cremiger Schaum erhalten wird.

### Beispiele

### Beispiele

Die folgende Tabelle enthält Beispielrezepturen für erfindungsgemäße Beispiele (Rezepturen), sowie Vergleichsbeispiele (Rezepturen).

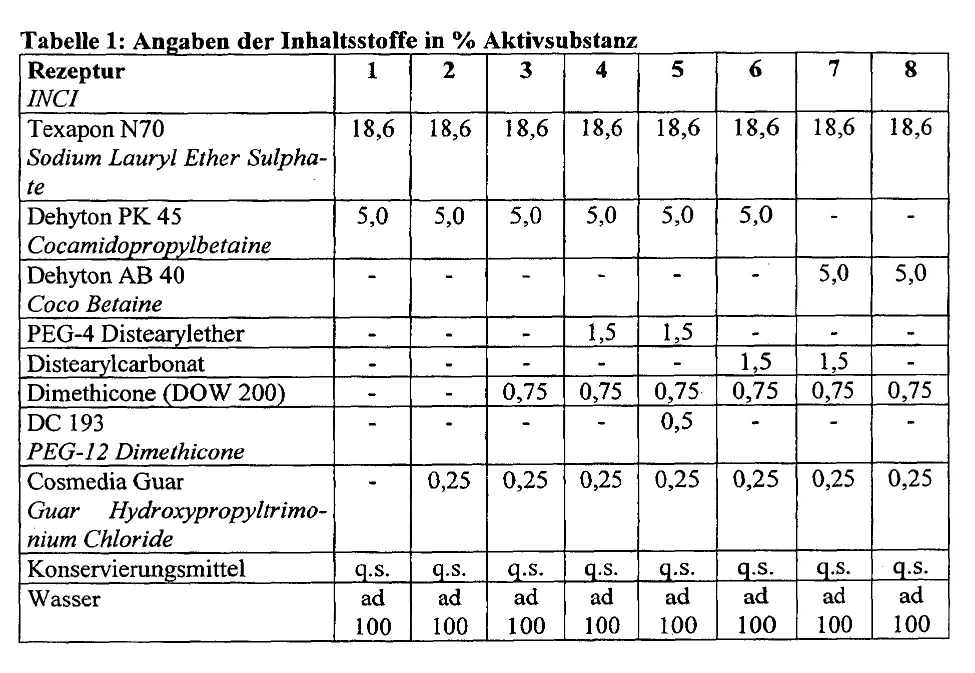

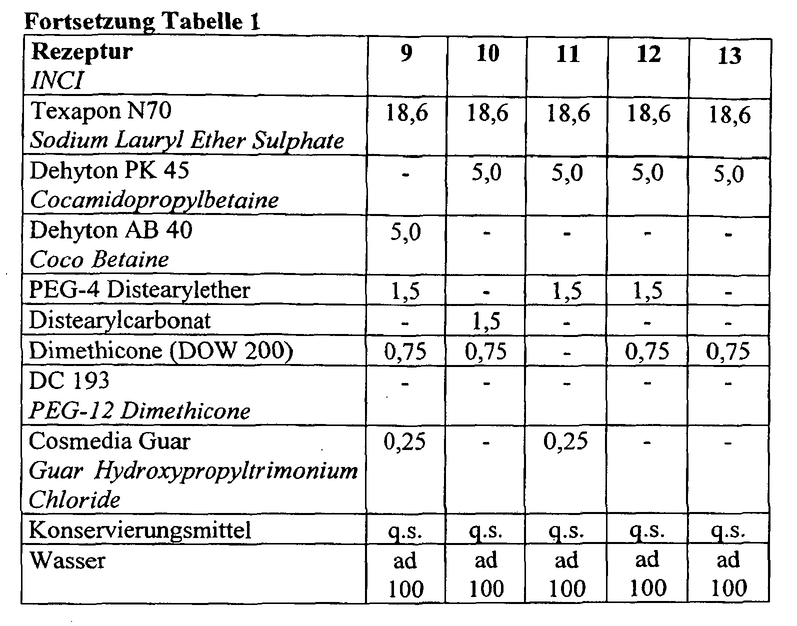

Für die o.g. Rezepturen 1 bis 13 wurde jeweils die Trocken- und Naßkämmbarkeit bestimmt.

Zur Ermittelung der Kämmarbeit wurden gebleichte Haarsträhnen mit dem zu untersuchenden Produkt (1g Produkt/1g Haar) behandelt, Einwirkzeit 5 min, dann ausgespült (Leitungswasser 38°C, 1 1/min). Anschließend wurden in einer speziellen Messapparatur die Kräfte bestimmt, die beim Kämmen der Haare auftreten. Aus den gemessenen Kraft-Weg-Verläufen wurde die Kämmarbeit durch Integration ermittelt. Der Quotient aus Kämmarbeit für unbehandelte und der Kämmarbeit an behandelten Haarsträhnen ergibt die sogenannte residuale Kämmarbeit. Jede Messung wurde an 20 Haarsträhnen durchgeführt.

Zur Bestimmung der Trockenkämmarbeit wurden 18 cm lange Haarsträhnen (2 g) verwendet, die vor der Messung in der abgeschlossenen Apparatur für 12 h klimatisiert wurden. Die Kämmung erfolgte mit Kämmen, die einen Zinkenabstand von 2,6 mm aufweisen. Für die Nasskämmarbeit wurden 15 cm Strähnen (1g) eingesetzt, die Kämmmessung erfolgte unmittelbar nach dem Ausspülen. Hier wurden Kämme mit einem Zinkenabstand von 0,7 mm eingesetzt. Um konstante Bedingungen zu gewährleisten, befand sich die gesamte Apparatur in einer Einhausung, in der eine Temperatur von 30°C und eine relative Luftfeuchte von 40% eingestellt wurde.

**Tabelle 2:**

| **Rezeptur** | Naß kämmarbeit **vorher** [mJ] | Naß kämmarbeit **nachher** [mJ] | Restkämmarbeit [%] |
|---|---|---|---|
| **1** | 104.41 | 108.4 | 103.82 |
| **2** | 108.51 | 100.54 | 92.66 |
| **3** | 104.24 | 76.85 | 73.72 |
| **4** | 100.32 | 61.11 | 60.09 |
| **5** | 108.02 | 53.33 | 49.37 |
| **6** | 106.44 | 52,88 | 49.68 |
| **7** | 105.77 | 53.07 | 50,17 |
| **8** | 105,33 | 66.63 | 63.26 |
| 9 | 102,44 | 62,08 | 60,60 |
| 10 | 100,87 | 66,35 | 65,78 |
| 11 | 107,61 | 81,20 | 75,45 |
| 12 | 101,02 | 65,43 | 64,76 |
| 13 | 107,93 | 83,61 | 77,46 |

| | | | |
|---|---|---|---|
| Die Messreihen sind zu 95% signifikant unterschieden in Bezug auf die Nasskämmarbeit vorher. | | | |

**Tabelle: 3**

| **Rezeptur** | Trockenkämmarbeit **vorher** [mJ] | Trockenkämmarbeit **nachher** [mJ] | Restkämm arbeit [%] |
|---|---|---|---|
| **1** | 55.21 | 52.81 | 95.65 |
| **2** | 49.51 | 41.9 | 84.62 |
| **3** | 56.65 | 37.81 | 66.74 |
| **4** | 57.31 | 24.32 | 42,43 |
| **5** | 54.56 | 17.54 | 32,14 |
| **6** | 48,55 | 21.33 | 43.93 |
| **7** | 52.02 | 24.06 | 46.26 |
| **8** | 57.42 | 36.30 | 63.21 |
| **9** | 54,77 | 23,63 | 43,14 |
| **10** | 52,79 | 26,77 | 50,71 |
| **11** | 56,37 | 42,81 | 75,94 |
| **12** | 49,94 | 24,68 | 49,41 |
| **13** | 56,41 | 42,75 | 75,78 |

| | | | |
|---|---|---|---|
| Die Messreihen sind zu 95% signifikant unterschieden in Bezug auf die Trockenkämmarbeit vorher. | | | |

Den Ergebnissen der Naß- und Trockenkämmbarkeit ist zu entnehmen, dass die erfindungsgemäßen Kombinationen eines Wachses mit Silikonen und kationischen Polymeren die stärkste Erniedrigung der Kämmarbeit bewirken (Messreihen 4 bis 7 und 9). Durch Vergleich der Ergebnisse der übrigen Meßreihen wird die synergistische Wirkung in bezug auf die Kämmarbeitserniedrigung der Kombinationen Tensid mit Polymer (2), Tensid mit Silikon (13), Tensid mit Polymer und Silikon (3 und 8) im Vergleich zu einer nur Tensid enthaltenden Zusammensetzung deutlich. Durch Zugabe eines Wachses zu einer Zubereitung enthaltend kationisches Polymer und Silikon wird die Kämmarbeit noch einmal deutlich vermindert.

## Patentansprüche

1. Kosmetische Zubereitung enthaltend
(a) mindestens einen Wachs ausgewählt aus der Gruppe, die gebildet wird von Dialkyl(en)alkoxyethern der Formel (I)
R¹-(OCₙH₂ₙ)ₓ-O-(CₘH₂ₘO)_{y}-R² (I)
in der R¹ und R² unabhängig voneinander für Alkyl- und/oder Alkylenreste mit 16 bis 36 Kohlenstoffatomen, n und m unabhängig voneinander für Zahlen von 2 bis 4 und x und y unabhängig voneinander für Zahlen von 0 bis 10 stehen, wobei die Summe von x und y 1 bis 10 beträgt,
und Dialkyl(en)carbonaten gemäß Formel (II)
R³O-CO-OR⁴ (II)
in der R³ und R⁴ unabhängig voneinander für gegebenenfalls hydroxyfunktionalisierteAlkyl- und/oder Alkenylreste mit 16 bis 36 Kohlenstoffatomen stehen, sowie Mischungen hiervon,
(b) mindestens ein Silikon,
(c) mindestens ein amphoteres und/oder zwitterionisches Tensid und
(d) mindestens ein kationisches Polymer.

2. Kosmetische Zubereitung gemäß Anspruch 1 enthaltend
(a) 0,3 bis 5 Gew.% mindestens eines Wachses ausgewählt aus der Gruppe, die gebildet wird von
Dialkyl(en)alkoxyethern der Formel (I)
R¹-(OCₙH₁ₙ)ₓ-O-(CₘH₂ₘO)_{y}-R² (I)
in der R1 und R2 unabhängig voneinander für Alkyl- und/oder Alkylenreste mit 16 bis 36 Kohlenstoffatomen, n und m unabhängig voneinander für Zahlen von 2 bis 4 und x und y unabhängig für Zahlen von 0 bis 10 stehen, wobei die Summe von x und y 1 bis 10 beträgt,
und Dialkyl(en)carbonaten gemäß Formel (II)
R³O-CO-OR⁴ (II)
in der R³ und R⁴ unabhängig voneinander für gegebenenfalls hydroxyfunktionalisierteAlkyl- und/oder Alkenylreste mit 16 bis 36 Kohlenstoffatomen stehen, sowie Mischungen hiervon,
(b) 0,2 bis 5 Gew.% mindestens eines Silikons,
(c) 1 bis 50 Gew.% mindestens eines amphoteren und/oder zwitterionischen Tensids und
(d) 0,1 bis 1 Gew.% mindestens eines kationischen Polymers.

3. Zubereitung gemäß wenigstens einem der Ansprüche 1 und 2, **dadurch gekennzeichnet, dass** als Tensid (c) Cocamidopropylbetain eingesetzt wird.

4. Zubereitung gemäß wenigstens einem der Ansprüche 1 bis 3, **dadurch gekennzeichnet, dass** die Komponente (d) ausgewählt ist aus der Gruppe, die gebildet wird von kationisch modifizierten Polyacrylaten, Polysacchariden, Polyacrylamiden oder einem beliebigen Gemisch dieser Polymere.

5. Zubereitung gemäß wenigstens einem der Ansprüche 1 bis 4, **dadurch** gekennzeichet, **dass** die Komponente (b) ausgewählt ist aus der Gruppe, die gebildet wird von
- Polyalkylsiloxanen,
- Polyalkylarylsiloxanen,
- Silikongummis,
- Silikonharzen und
- organisch modifizierten Silikonen.

## Claims

1. A cosmetic preparation comprising
(a) at least one wax selected from the group which is formed from dialkyl(ene)alkoxy ethers of the formula (I)
R¹-(OCₙH₂ₙ)ₓ-O-(CₘH₂ₘO)_{y}-R² (I)
in which R¹ and R², independently of one another, are alkyl and/or alkylene radicals having 16 to 36 carbon atoms, n and m, independently of one another, are numbers from 2 to 4 and x and y, independently of one another, are numbers from 0 to 10, where the sum of x and y is 1 to 10,
and dialkyl(ene) carbonates according to formula (II)
R³O-CO-OR⁴ (II)
in which R³ and R⁴, independently of one another, are optionally hydroxyfunctionalized alkyl and/or alkenyl radicals having 16 to 36 carbon atoms, and mixtures thereof,
(b) at least one silicone,
(c) at least one amphoteric and/or zwitterionic surfactant and
(d) at least one cationic polymer.

2. The cosmetic preparation according to claim 1, comprising
(a) 0.3 to 5% by weight of at least one wax selected from the group which is formed from dialkyl(ene)alkoxy ethers of the formula (I)
R¹-(OCₙH₂ₙ)ₓ-O-(CₘH₂ₘO)_{y}-R² (I)
in which R¹ and R², independently of one another, are alkyl and/or alkylene radicals having 16 to 36 carbon atoms, n and m, independently of one another, are numbers from 2 to 4 and x and y are independently numbers from 0 to 10, where the sum of x and y is 1 to 10,
and dialkyl(ene) carbonates according to formula (II)
R³O-CO-OR⁴ (II)
in which R³ and R⁴, independently of one another, are optionally hydroxyfunctionalized alkyl and/or alkenyl radicals having 16 to 36 carbon atoms, and mixtures thereof,
(b) 0.2 to 5% by weight of at least one silicone,
(c) 1 to 50% by weight of at least one amphoteric and/or zwitterionic surfactant and
(d) 0.1 to 1% by weight of at least one cationic polymer.

3. The preparation according to at least one of claims 1 and 2, wherein cocamidopropylbetaine is used as surfactant (c).

4. The preparation according to at least one of claims 1 to 3, wherein the component (d) is selected from the group which is formed form cationically modified polyacrylates, polysaccharides, polyacrylamides or any desired mixture of these polymers.

5. The preparation according to at least one of claims 1 to 4, wherein the component (b) is selected from the group which is formed from
- polyalkylsiloxanes,
- polyalkylarylsiloxanes,
- silicone gums,
- silicone resins and
- organically modified silicones.

## Revendications

1. Préparation cosmétique contenant
(a) au moins une cire choisie dans le groupe qui est formé par les dialkyl(én)alcoxyéthers de formule (I)
R¹-(OCₙH₂ₙ)ₓ-O-(CₘH₂ₘO)_{y}-R² (I)
dans laquelle R¹ et R² représentent, indépendamment l'un de l'autre, des radicaux alkyle et/ou alkylène comprenant 16 à 36 atomes de carbone, n et m représentent, indépendamment l'un de l'autre, des nombres de 2 à 4 et x et y représentent, indépendamment l'un de l'autre, des nombres de 0 à 10, la somme de x et y valant 1 à 10,
et des dialkyl(ène)carbonates selon la formule (II)
R³O-CO-OR⁴ (II)
dans laquelle R³ et R⁴ représentent, indépendamment l'un de l'autre, des radicaux alkyle et/ou alcényle, le cas échéant fonctionnalisés par hydroxy, comprenant 16 à 36 atomes de carbone, ainsi que leurs mélanges,
(b) au moins une silicone,
(c) au moins un tensioactif amphotère et/ou zwittérionique et
(d) au moins un polymère cationique.

2. Préparation cosmétique selon la revendication 1, contenant
(a) 0,3 à 5 % en poids d'au moins une cire choisie dans le groupe qui est formé par les dialkyl(én)alcoxyéthers de formule (I)
R¹-(OCₙH₂ₙ)ₓ-O-(CₘH₂ₘO)_{y}-R² (I)
dans laquelle R¹ et R² représentent, indépendamment l'un de l'autre, des radicaux alkyle et/ou alkylène comprenant 16 à 36 atomes de carbone, n et m représentent, indépendamment l'un de l'autre, des nombres de 2 à 4 et x et y représentent, indépendamment l'un de l'autre, des nombres de 0 à 10, la somme de x et y valant 1 à 10,
et des dialkyl(ène)carbonates selon la formule (II)
R³O-CO-OR⁴ (II)
dans laquelle R³ et R⁴ représentent, indépendamment l'un de l'autre, des radicaux alkyle et/ou alcényle, le cas échéant fonctionnalisés par hydroxy, comprenant 16 à 36 atomes de carbone, ainsi que leurs mélanges,
(b) 0,2 à 5% en poids d'au moins une silicone,
(c) 1 à 50 % en poids d'au moins un tensioactif amphotère et/ou zwittérionique et
(d) 0,1 à 1% en poids d'au moins un polymère cationique.

3. Préparation selon au moins l'une quelconque des revendications 1 et 2, **caractérisée en ce qu'**on utilise, comme tensioactif (c), de la cocoamidopropylbétaïne.

4. Préparation selon au moins l'une quelconque des revendications 1 à 3, **caractérisée en ce que** le composant (d) est choisi dans le groupe formé par les polyacrylates, les polysaccharides, les polyacrylamides modifiés de manière cationique ou un mélange quelconque de ces polymères.

5. Préparation selon au moins l'une quelconque des revendications 1 à 4, **caractérisée en ce que** le composant (b) est choisi dans le groupe formé par
- les polyalkylsiloxanes,
- les polyalkylarylsiloxanes,
- les gommes de silicone,
- les résines de silicone et
- les silicones organiquement modifiées.
